# EUROPEAN PATENT APPLICATION

(11) **EP 1 967 154 A1**
(43) Date of publication of application: **10.09.2008**
(21) Application number: 08003652.8
(22) Date of filing: 28.02.2008
(51) Int. Cl.: A61B 18/08

(54) **Needle or catheter for the termal coagulation of blood vessels**

(30) Priority: 02.03.2007 BE 200700092; 01.10.2007 BE 200700468
(71) Applicant: F-CARE SYSTEMS, naamloze vennootschap, 2630 Aartselaar (BE)
(72) Inventor: Devers, Rudi Madeleine Fernand, 2650 Edegem (BE)
(74) Representative: Donné, Eddy

(57) **Abstract**

Needle or catheter for thermally coagulating blood vessels, which mainly consists of an elongated body (2) with a proximal end (3) and a distal end (4), whereby a tip (6) in a conductive material is provided at the distal end (4), whereby a first conductor (7) which makes contact with the tip (6) extends between the distal end (4) and the proximal end (3), and whereby the outside of the elongated body (2) is provided over at least part of its length, but not at the tip (6), with an insulation (10), characterized in that the needle or the catheter (1) comprises a second conductor (8) which extends, isolated from the first conductor (7), as of the distal end (4) up to the proximal end (3) and which makes contact with the tip (6).

## Description

The present invention concerns a needle or a catheter for the thermal coagulation of blood vessels.

Thermally coagulating blood vessels is a known technique for treating varicose veins and the like.

FR 2,816,217 and 0,983,751 describe a method for removing varicose veins or the like by means of thermo coagulation, whereby a needle is made to penetrate with its tip in a vein to be removed, after which the tip of the needle is heated by means of radio-frequency waves, as a result of which the walls of the vein coagulate and the vein is locally sealed such that the bleeding stops, after which the vein is disintegrated by the body within a couple of days or weeks.

Known needles that are applied for the thermal coagulation of blood vessels mainly consist of an elongated body with a proximal end and a distal end, whereby a tip made of a conductive material is provided on the distal end, whereby a conductor which makes contact with the tip extends between the distal end and the proximal end and whereby the outside of the needle is provided with an insulation over at least part of its length, but not at the tip.

The radio-frequency waves heat the tip of said needle, as a result of which it can be applied for the thermal coagulation of blood vessels.

A disadvantage of these known needles, however, is that the radio-frequency waves which reach the tip of the needle propagate within the patient's body, such that apart from the local heating by the tip of the needles, heat is also generated in other locations in the patient's body, which is unwished-for.

The above-mentioned disadvantageous effect becomes more significant as the size of the blood vessels being treated increases, as the treatment time or the amplitude of the radio-frequency waves increases.

For the reasons mentioned above, the thermal coagulation technique for blood vessels is restricted to blood vessels having a maximum diameter of 1 mm for cosmetic reasons.

The present invention aims to treat blood vessels having a diameter of more than 1 mm by means of thermal coagulation as well.

The use of catheters for treating varicose veins is known. A catheter is applied in particular when treating varicose veins that are difficult to reach by means of conventional needles.

To this end, the invention concerns a needle or a catheter which mainly consists of an elongated body having a proximal end and a distal end, whereby a tip made of a conductive material is provided at the distal end, whereby a first conductor which makes contact with the tip extends between the distal end and the proximal end and whereby the outside of the elongated body is provided with insulation over at least part of its length, but not at the tip, characterized in that the needle or the catheter comprises a second conductor which extends, isolated from the first conductor, as of the distal end up to the proximal end and which makes contact with the tip.

An advantage of the present invention is that providing the second conductor and bringing it into contact with the tip makes it possible to divert the radio-frequency waves, such that they will not propagate within the patient's body.

Another advantage of the invention is that the needle makes it possible to treat blood vessels having a diameter of more than 2 mm or even 3 mm without this having any disadvantageous effect on the patient.

In order to better explain the characteristics of the present invention, a preferred embodiment of a needle and of a catheter according to the invention for thermally coagulating blood vessels is described hereafter with reference to the accompanying drawings, in which:
figure 1 represents a needle according to the invention;
figure 2 represents a section according to line II-II in figure 1;
figure 3 represents the part indicated by F3 in figure 2 to a larger scale;
figure 4 represents a catheter according to the invention;
figure 5 represents a section according to line V-V in figure 4;
figure 6 represents the part indicated by F6 in figure 5 to a larger scale.

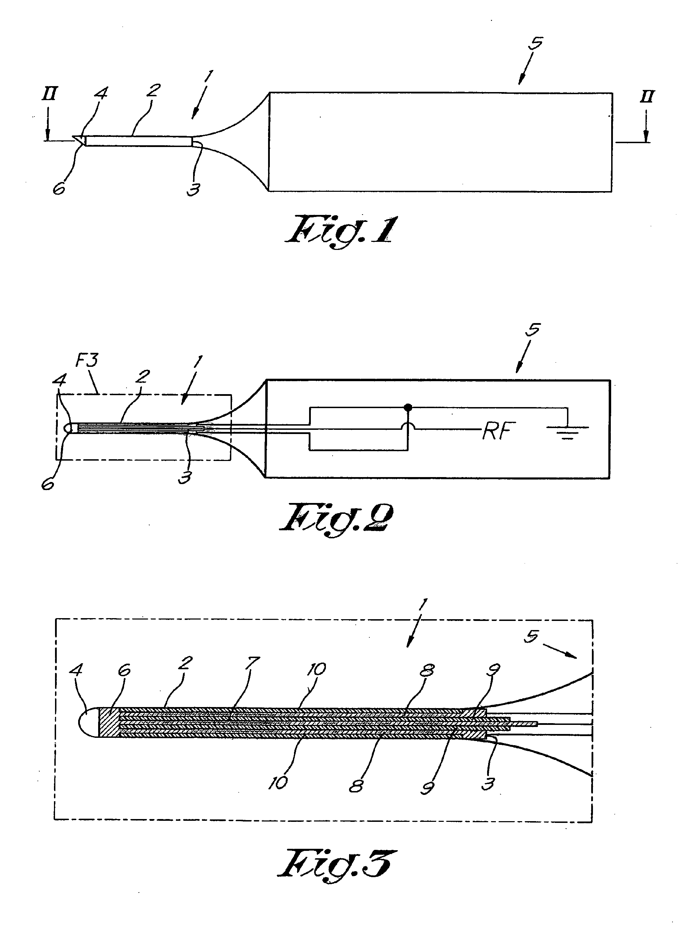

Figures 1 and 2 represent a needle 1 according to the invention which mainly consists of an elongated body 2 having a proximal end 3 and a distal end 4. By the proximal end is hereby meant the far end of the needle 1 which is directed towards a user of the needle 1 when used correctly, whereas the distal end 4 is the far end which penetrates into the patient's body when in use.

The needle 1 is preferably provided in a handle 5 with its proximal end.

It clear that the needle 1 is preferably provided in the handle in a removable manner, such that the handle can be re-used, whereas the needle is removed after each use.

At the distal end 4, the needle 1 opens in a preferably sharp tip 6.

This tip 6 is made of a material which conducts radio-frequency waves and which heats up under the influence of appropriate radio-frequency waves, an example of such a material being nickel.

According to the invention, and as is represented in greater detail in figure 3, the needle 1 comprises at least two conductors 7 and 8 for radio-frequency waves, extending as of the distal end 4 up to the proximal end 3, which conductors 7 and 8 are isolated from one another and are both in conductive contact with the tip 6.

In the given embodiment, the needle 1 is layered, with the first conductor 7 in the centre as the core of the elongated body 2, and coaxially around it a first insulating layer 9, with the second conductor 8 coaxially around it and, coaxially around the latter, a second outer insulating layer 10.

The conductors 7 and 8 are conductors for radio-frequency waves and they are preferably made of nickel. The insulating layers 9 and 10 are made of a material which insulates against radio-frequency waves, such as Teflon or another insulating material.

One of the conductors 7 or 8, in this case the first conductor 7, is connected to a generator 11 of radio-frequency waves at its proximal end, whereas the other conductor, in this case the second conductor 8, is connected to a grounding 12.

The connection between the conductors 7 and 8 and the generator, the grounding respectively, is preferably realized by means of an appropriate seat in the handle 5, such that the needle can be replaced after each use and the handle can be further used in combination with a new needle.

As a generator 11 for radio-frequency waves, model TC3000 of Fcare Systems can be used, for example, which can generate radio-frequency waves having a frequency between 3 megahertz (MHz) and 5 MHz.

The above-mentioned generator 11 is in this case provided in the handle 5, but it can also be external, whereby the generator 11 is connected to one of the conductors 7 or 8 by means of a guide provided for example through the handle 5.

The generator 11 is preferably provided with means which make it possible to operate it in a simple manner, for example by means of a foot pedal which activates the generator as it is stepped on. The generator further preferably comprises a control unit and a control panel, not represented in the figures, which make it possible to set the generator 11 at a desired frequency and a desired interval. Thus, when the foot pedal is stepped on, the generator will generate waves having the set frequency during the set interval.

The grounding 12, which is only represented schematically in the figures, may consist of a conductor for radio-frequency waves which is connected to the ground or, via the grounding of the generator 11, to the grounding of the supply network.

The use of a needle according to the invention is simple and as follows.

In order to treat a varicose vein or the like, the needle 1 is provided in a patient with its distal end 4 until the tip 6 of the needle 1 makes contact with the varicose vein to be treated. Next, the generator 11 for radio-frequency waves is activated, as a result of which the tip 6 is heated to some 60 to 70°C, such that the vein coagulates, after which the generator 11 is deactivated and the needle is removed from the body. In order to heat the tip 6, radio-frequency waves having a wavelength between 3 MHz and 5 MHz are preferably used.

Thanks to the thermal coagulation, the treated vein is locally sealed and it will be rejected by the patient's body within a few days or weeks.

As, preferably, only the tip 6 of the needle is not insulated, only the tissue which makes contact with the tip will be heated during a treatment. The radio-frequency waves which propagate through the needle 1 will moreover be diverted again via the needle 1 to the grounding 12, as a result of which these waves cannot or can only be transmitted to the patient's body up to a very limited extent. Hence, possible side-effects for the patient caused by the radio-frequency waves, which propagate in his/her body, are excluded.

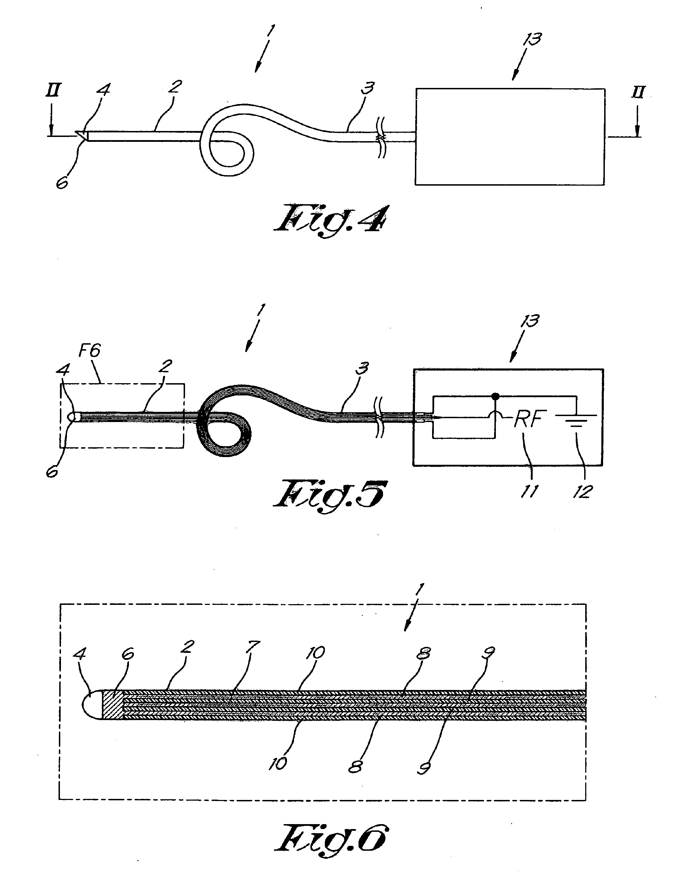

Figures 4 and 5 represent a catheter 1 according to the invention which mainly consists of an elongated flexible body 2 with a proximal end 3 and a distal end 4 which penetrates into the patient's body when in use.

The catheter 1 is preferably provided with its proximal end 3 in a control unit 13.

It is clear that the catheter 1 is preferably provided in the control unit 13 in a removable manner, such that it can be re-used, whereas the catheter 1 can be replaced on a regular basis.

At the distal end 4, the catheter 1 opens in a preferably sharp tip 6.

This tip 6 is made of a material such as for example nickel, which conducts radio-frequency waves and which heats up under the influence of suitable radio-frequency waves.

According to the invention and as represented in more detail in figure 6, the catheter 1 comprises at least two conductors 7 and 8 for radio-frequency waves, which extend as of the distal end 4 up to the proximal end 3, which conductors 7 and 8 are isolated from one another and are both in conductive contact with the tip 6.

In the given embodiment, the catheter 1 is layered with, as a core of the elongated body 2, the first conductor 7 in the centre, a first insulating layer 9 provided coaxially around it, the second conductor 8 provided coaxially around it, and a second, outer insulation layer 10 provided coaxially round the latter.

The conductors 7 and 8 are conductors for radio-frequency waves and they are preferably made of nickel. The insulating layers 9 and 10 are made of a material which insulates against radio-frequency waves, such as Teflon or another insulating material.

One of the conductors 7 or 8, in this case the first conductor 7, is connected to a generator 11 for radio-frequency waves at its proximal end, whereas the other conductor, in this case the second conductor 8, is connected to a grounding 12.

The connection between the conductors 7 and 8 and the generator, the grounding respectively, is preferably provided for by means of an appropriate seat in the control unit 13.

As a generator 11 of radio-frequency waves, also model TC3000 of Fcare Systems could be used in this case, for example, which can generate radio-frequency waves having a frequency between 3 megahertz (MHz) and 5 MHz.

The above-mentioned generator 11 is in this case provided in the control unit 13, but it may also be external, whereby the generator 11 is connected to one of the conductors 7 or 8 by means of a conductor provided for example through the control unit 13.

Preferably, the generator 11 is provided with means which make it possible to control it in a simple manner, for example by means of foot pedal which makes it possible to activate the generator when being stepped on. The generator 11 further preferably comprises a control unit and a control panel, not represented in the figures, which make it possible to set the generator 11 at a desired frequency and at a desired interval. Consequently, when the foot pedal is being stepped on, the generator will generate waves having the set frequency during the set interval.

The grounding 12, which is only schematically represented in the figures, may consist of a conductor for radio-frequency waves which is connected to the ground or, via the grounding of the generator 11, to the grounding of the supply network.

The use of a catheter 1 according to the invention is simple and, save for the application of the catheter in the varicose vein, corresponds entirely to the use of a needle 1 according to the invention.

The present invention is by no means restricted to the embodiment described above and represented in the figures; on the contrary, such a needle or a catheter according to the invention for coagulating blood in blood vessels can be made according to many different variants while still remaining within the scope of the invention.

## Claims

1. Needle or catheter for thermally coagulating blood vessels, which mainly consists of an elongated body (2) with a proximal end (3) and a distal end (4), whereby a tip (6) in a conductive material is provided at the distal end (4), whereby a first conductor (7) which makes contact with the tip (6) extends between the distal end (4) and the proximal end (3), and whereby the outside of the elongated body (2) is provided over at least part of its length, but not at the tip (6), with an insulation (10), **characterized in that** the needle or the catheter (1) comprises a second conductor (8) which extends, isolated from the first conductor (7), as of the distal end (4) up to the proximal end (3) and which makes contact with the tip (6).

2. Needle or catheter according to claim 1, **characterized in that** the above-mentioned conductors (7-8) conduct radio-frequency waves and **in that** one of the above-mentioned conductors (7-8) is connected to a generator (11) of radio-frequency waves at its proximal end, and **in that** the other conductor (8-7) is connected to a grounding (12) at its proximal end.

3. Needle or catheter according to claim 1 or 2, **characterized in that** the second conductor (8) is provided concentrically round the first conductor (7), and **in that** these conductors (7-8) are separated from one another by an insulating layer (9) extending between both conductors (7-8) and whereby, round the second conductor (8), is provided a second insulating layer (10).

4. Needle or catheter according to any one of the preceding claims, **characterized in that** the first conductor (7) forms the core of the elongated body (2).

5. Needle or catheter according to any one of the preceding claims, **characterized in that** the conductors (7-8) are made of nickel.

6. Needle or catheter according to claim 3, **characterized in that** at least the second insulating layer (10) is made of Teflon or another insulating material.

7. Needle or catheter according to any one of the preceding claims, **characterized in that** the proximal end (3) of the elongated body (2) is provided in a handle (5).

8. Needle or catheter according to any one of the preceding claims, **characterized in that** it is provided with means which make it possible to activate and deactivate the generator (11) of radio-frequency waves.

9. Needle or catheter according to claim 8, **characterized in that** the above-mentioned means consist of a foot pedal.

10. Needle or catheter according to any one of the preceding claims, **characterized in that** the tip (6) is sharp.
